Europäisches Patentamt

European Patent Office

Office européen des brevets

(11) Veröffentlichungsnummer: **0 337 262**
**A1**

## EUROPÄISCHE PATENTANMELDUNG

(21) Anmeldenummer: 89105923.0

(22) Anmeldetag: 05.04.89

(51) Int. Cl.⁴: **C07C 53/122 , C07C 53/124 , C07C 53/126 , C07C 55/10 , C07C 51/48**

(30) Priorität: 14.04.88 DE 3812403

(43) Veröffentlichungstag der Anmeldung:
18.10.89 Patentblatt 89/42

(84) Benannte Vertragsstaaten:
BE CH DE FR GB IT LI NL

(71) Anmelder: **BASF Aktiengesellschaft**
**Carl-Bosch-Strasse 38**
**D-6700 Ludwigshafen(DE)**

(72) Erfinder: **Samel, Ulf-Rainer, Dr.**
**Rosenstrasse 11**
**D-6704 Mutterstadt(DE)**
Erfinder: **Bieg, Walter**
**Trifelsstrasse 32**
**D-6718 Gruenstadt(DE)**
Erfinder: **Broellos, Klaus, Dr.**
**Floriansring 10a**
**D-6104 Seeheim-Jugenheim(DE)**
Erfinder: **Irnich, Rudolf, Dr.**
**In den Hahndornen 2**
**D-6719 Bobenheim(DE)**

(54) Verfahren zur Rückgewinnung und Wiederverwendung von Nickel, Cobalt und Rhodium aus Gemischen, die aliphatische Carbonsäuren enthalten.

(57) Verfahren zur Rückgewinnung und Wiederverwendung von Nickel, Cobalt oder Rhodium aus Gemischen, die aliphatische Mono- und Dicarbonsäuren mit 3 bis 10 C-Atomen enthalten und die man durch Hydrocarboxylierung von Ethylen in Gegenwart von Nickel, Cobalt oder Rhodium enthaltenden Katalysatoren erhält, dadurch gekennzeichnet, daß man aus dem Gemisch destillativ die Propionsäure abtrennt, den Rückstand mit Wasser extrahiert und den die Metallsalze enthaltenden Extrakt erneut in die Hydrocarboxylierung einsetzt.

EP 0 337 262 A1

## Verfahren zur Rückgewinnung und Wiederverwendung von Nickel, Cobalt und Rhodium aus Gemischen, die aliphatische Carbonsäuren enthalten

Die vorliegende Erfindung betrifft ein Verfahren zur Rückgewinnung und Wiederverwendung von Nickel, Cobalt und Rhodium aus Gemischen, die aliphatische Carbonsäuren enthalten.

Nickel, Cobalt und Rhodium sind in der Technik als Metalle oder in ihren Verbindungen häufig benutzte Katalysatoren. Typische Reaktionen, für die sie verwendet werden, sind z.B. Hydrierungen, Hydrocarboxylierungen, aber auch Crackreaktionen. Die genannten Metalle kommen dabei sowohl in gelöster Form, als Salze anorganischer oder organischer Säuren, als auch in metallischer Form auf Trägermaterialien zum Einsatz. Beispielsweise durch Abrieb finden sich diese Metalle im Rohprodukt von den oben genannten Umsetzungen wieder.

Die Aufarbeitung des metallhaltigen Rückstands des Rohprodukts ist im allgemeinen aufwendig, zumal der Metallgehalt häufig nur gering ist.

Aus der EP-A-106 271 ist bekannt, daß man solche metallhaltigen Rückstände in einen Dünnschichtverdampfer einengen kann. Der eingeengte Rest wird nach dem Wiederauflösen in Carbonsäure erneut in der Synthese eingesetzt. Nach diesem Verfahren werden jedoch die Schwersieder immer weiter mitangereichert.

Der Erfindung lag daher die Aufgabe zugrunde, ein Verfahren zu entwickeln, das den metallhaltigen Rückstand anreichert und den überwiegenden Teil der Schwersieder abtrennt.

Demgemäß wurde ein neues und verbessertes Verfahren zur Rückgewinnung und Wiederverwendung von Nickel, Cobalt oder Rhodium aus Gemischen, die aliphatische Mono- und Dicarbonsäuren mit 3 bis 10 C-Atomen enthalten und die man durch Hydrocarboxylierung von Ethylen in Gegenwart von Nickel, Cobalt oder Rhodium enthaltenden Katalysatoren erhält, gefunden, welches dadurch gekennzeichnet ist, daß man aus dem Gemisch destillativ die Propionsäure abtrennt, den Rückstand mit Wasser extrahiert und den die Metallsalze enthaltenden Extrakt erneut in die Hydrocarboxylierung einsetzt.

Die Ausgangsgemische für die Rückgewinnung von Nickel, Cobalt oder Rhodium nach dem erfindungsgemäßen Verfahren sind Rückstände, die bei einer in US-A-3 835 185 beschriebenen Hydrocarboxylierung von Ethylen in Gegenwart von Katalysator aus den o.g. Metallen anfallen.

Solch ein Gemisch wird z.B. bei der Propionsäuresynthese als Destillationsrückstand erhalten. Als Nebenprodukt treten Mono- und Dicarbonsäuren z.B. gesättigte Monocarbonsäuren wie Buttersäuren, Valeriansäure, Hexansäuren, Heptansäuren oder gesättigte Dicarbonsäuren wie z.B. Bernsteinsäure auf, sowie die o.g. Metalle bzw. die Salze dieser Metalle mit den genannten Säuren.

Die metallsalzhaltigen Säurerückstände können beispielsweise in einer Extraktionskolonne mit Wasser extrahiert werden. Zur besseren Durchmischung kann die Extraktion pulsierend und auf erhöhter Temperatur von 35 bis 100°C, vorzugsweise bei 40 bis 70°C betrieben werden. Die zur Extraktion benutzte Wassermenge liegt zwischen 10 und 200 Gew.% der zu extrahierenden Rückstandsmenge, vorzugsweise bei 50 bis 100 Gew.%.

Der wäßrige Extrakt enthält das Nickel, Cobalt oder Rhodium quantitativ und ca. 5 bis 10 Gew.% der in die Extraktion eingesetzten organischen Komponenten. Dieser kann direkt in der Synthese wiederverwendet werden.

Die anfallende organische Phase ist nickelfrei und enthält ca. 5 bis 10 Gew.% Wasser.

Beispiel

50 l/h eines Rückstandgemisches, bestehend aus

| Propionsäure | 8 Gew.% |
|---|---|
| n-, iso-Buttersäure | 1 Gew.% |
| n-Valeriansäure | 28 Gew.% |
| iso-Valeriansäure | 24 Gew.% |
| 2-Methyl-Buttersäure | 4 Gew.% |
| n-Hexansäure | 2 Gew.% |
| Dimethylvaleriansäure | 20 Gew.% |
| n-Heptansäure | 5 Gew.% |
| Nickel | 0,4 Gew.% |
| sonstigen Verbindungen | 7,6 Gew.% |

wird kontinuierlich in einer pulsierten Extraktionskolonne mit 25 l Wasser/h extrahiert.

Das Raffinat hat folgende Zusammensetzung:

| Propionsäure | 5 Gew.% |
|---|---|
| Schwersieder | 85 Gew.% |
| Wasser | 10 Gew.% |
| Nickel | 0,00 Gew.% |

Der Extrakt hat folgende Zusammensetzung:

| Propionsäure | 3 Gew.% |
|---|---|
| Schwersieder | 13 Gew.% |
| Wasser | 83 Gew.% |
| Nickel | 1 Gew.% |

## Ansprüche

1. Verfahren zur Rückgewinnung und Wiederverwendung von Nickel, Cobalt oder Rhodium aus Gemischen, die aliphatische Mono- und Dicarbonsäuren mit 3 bis 10 C-Atomen enthalten und die man durch Hydrocarboxylierung von Ethylen in Gegenwart von Nickel, Cobalt oder Rhodium enthaltenden Katalysatoren erhält, dadurch gekennzeichnet, daß man aus dem Gemisch destillativ die Propionsäure abtrennt, den Rückstand mit Wasser extrahiert und den die Metallsalze enthaltenden Extrakt erneut in die Hydrocarboxylierung einsetzt.

2. Verfahren nach Anspruch 1, dadurch gekennzeichnet, daß man Nickel aus Gemischen zurückgewinnt, die Propionsäure enthalten und die man durch Hydrocarboxylierung von Ethylen in Gegenwart von Nickel enthaltenden Katalysatoren erhält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-C- 950 549 (BADISCHE ANILIN- & SODA-FABRIK) <br> * Anspruch; Seiten 2-3, Beispiel * <br> --- | 1-2 | C 07 C 53/122 <br> C 07 C 53/124 <br> C 07 C 53/126 <br> C 07 C 55/10 <br> C 07 C 51/48 |
| Y | DE-C- 951 983 (BADISCHE ANILIN- & SODA-FABRIK) <br> * Ansprüche 1-3 * <br> --- | 1-2 | |
| Y | EP-A-0 032 524 (CHEMISCHE WERKE HÜLS AG) <br> * Ansprüche 1-2; Seite 5, Zeile 14 - Seite 6, Zeile 24 * <br> --- | 1-2 | |
| A | US-A-4 284 523 (DARIN et al.) <br> * Anspruch 1 * <br> ----- | 1 | |

|  |  |  | RECHERCHIERTE SACHGEBIETE (Int. Cl.4) |
|---|---|---|---|
|  |  |  | C 07 C 53/00 <br> C 07 C 55/00 <br> C 07 C 51/00 <br> C 07 C 69/00 <br> C 07 C 67/00 |

Der vorliegende Recherchenbericht wurde für alle Patentansprüche erstellt

| Recherchenort | Abschlußdatum der Recherche | Prüfer |
|---|---|---|
| DEN HAAG | 05-07-1989 | KLAG M.J. |